Europäisches Patentamt

European Patent Office

Office européen des brevets

(11) Publication number: **0 251 146**
**A2**

# (12) EUROPEAN PATENT APPLICATION

(21) Application number: **87108999.1**

(22) Date of filing: **23.06.87**

(51) Int. Cl.⁴: **A61K 7/18**

(30) Priority: **27.06.86 NO 862611**

(43) Date of publication of application:
**07.01.88 Bulletin 88/01**

(84) Designated Contracting States:
**AT BE CH DE ES FR GB GR IT LI LU NL SE**

(71) Applicant: **Blendax-Werke R. Schneider GmbH & Co.**
**Rheinallee 88**
**D-6500 Mainz(DE)**

(72) Inventor: **Rolla, Gunnar, Prof. Dr.**
**Krags vei 13**
**N-0391 Oslo 3(NO)**
Inventor: **Assev, Synnove**
**Theodor Lovstadsvei 16**
**N-0286 Oslo 2(NO)**
Inventor: **Scheie, Anne Aa.**
**Grakamvn 19 c**
**N-0369 Oslo 3(NO)**

(54) **Composition for oral hygiene.**

(57) The invention relates to compositions for oral hygiene such as toothpastes, mouthwashes, chewing gums, etc. containing a synergistic 3-component mixture of a fluorine compound, e.g. sodium fluoride and (or) sodium monofluorophosphate, a zinc ions releasing compound, e.g. zinc chloride, zinc acetate and (or) zinc citrate, and xylitol. This mixture shows an improved activity against Streptococcus sp., reduces dental plaque formation and pH decrease after sugar consumption.

EP 0 251 146 A2

## COMPOSITION FOR ORAL HYGIENE

The present invention relates to a composition for oral hygiene, especially a tooth and mouth care agent having a dental plaque preventing or diminishing and thus caries-prophylactic activity, containing a synergistic mixture of at least one fluorine compound, at least one zinc ions releasing zinc compound, and xylitol.

It is well known that fluorides affect bacterial metabolism and the ability of bacteria to multiply, and different fluoride salts are therefore used in preparations designed to prevent dental caries. Fluoride reduces the bacterial acid production by inhibiting enolase.

It is also well known that zinc, particularly as zinc chloride or zinc citrate, reduces bacterial growth and acid production, probably by displacement of cations that are of significance for the enzymatic activity.

Finally it is known that pentitols and hexitols, especially xylitol, inhibit glucose metabolism in oral streptococci.

It is believed that xylitol causes this inhibition by competition for available PEP (phosphoenol pyruvate) or by a direct inhibition of the glycolytic enzyme phosphofructokinase.

However, although it has been tried to incorporate these substances into compositions for oral hygiene such as toothpastes and mouthwashes and also combinations of e.g. zinc citrate or zinc chloride and fluorine compounds have been used for this purpose, a satisfying effect as to the prevention of dental plaque and consequently reduction of caries could not be obtained by those preparations.

Surprisingly, it has now been found, and this is the object of our invention, that a combination of all three compounds shows a synergistic effect compared to the action of the single components and their well-known two component mixtures.

This synergistic effect is shown by the following experiments:

Streptococcus mutans OMZ 176 was grown in a batch culture of brain heart infusion broth with 1 % xylitol, 100 ppm of NaF and 0.5 mM of $ZnCl_2$ individually or in combination at pH 6.7 and 37° C. To 2 ml samples were added 0.2 ml $14_C$ -glucose (2.2 × $10^6$ cpm/ml, 295 mCi/mMol, NEN), and samples of 100 ml were withdrawn and applied on filter paper discs after suitable time intervals.

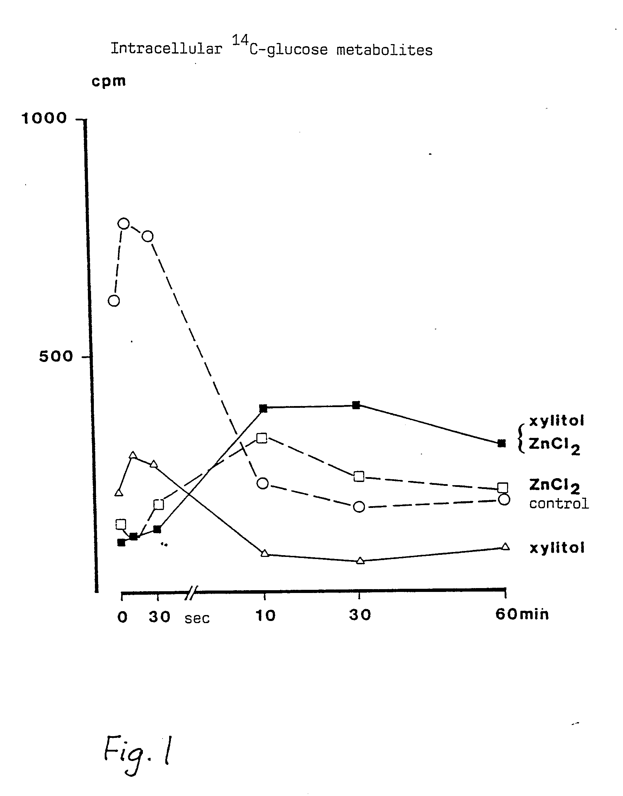

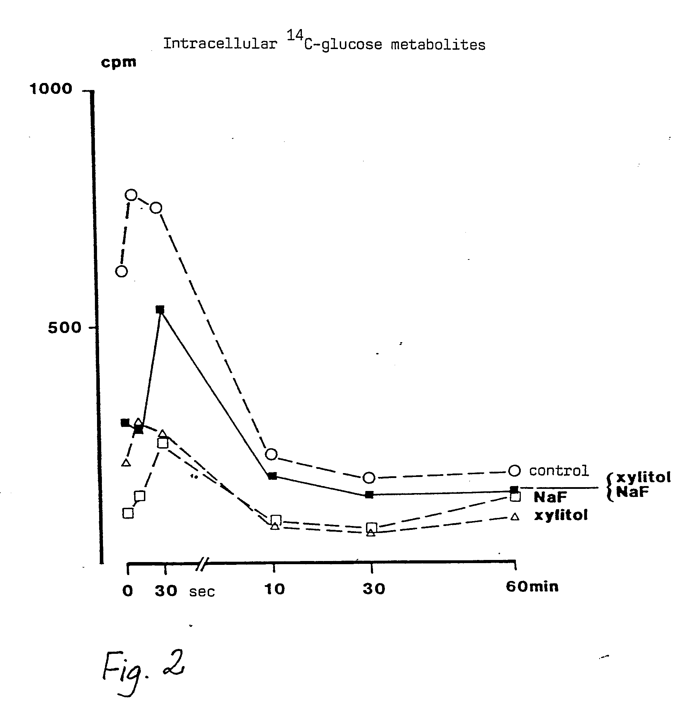

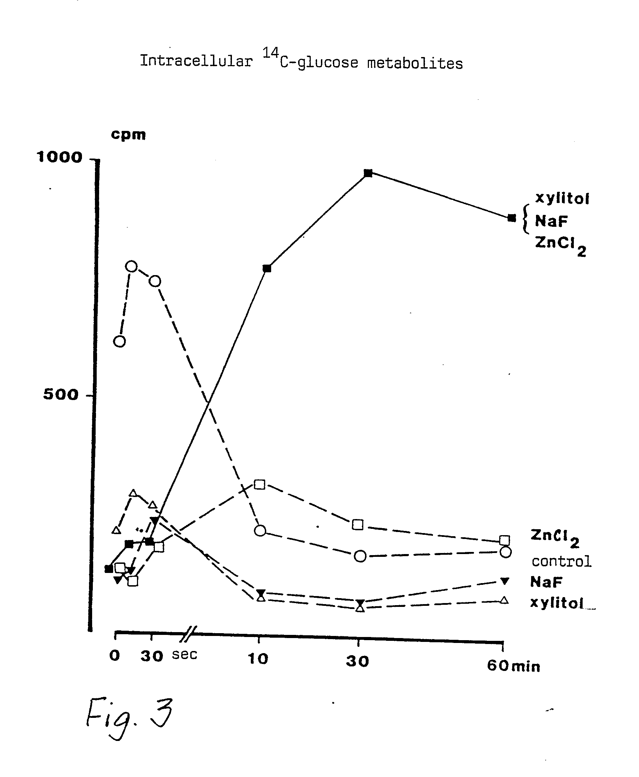

The bacteria were fixed to the filter paper by application of absolute ethanol; whereby the free glucose was eliminated. The filter paper discs were subsequently washed twice with 75 % ethanol, scintillation liquid was added, and the samples were subjected to scintillation counting. The results of these experiments can be seen from figures 1 to 3. It is clearly demonstrated that zinc ions, xylitol, or fluoride individually or these ingredients applied in two-component combinations generally lead to a reduced uptake of glucose compared to the control group.

Concerning the combination of zinc, xylitol, and fluoride, the uptake of glucose was delayed and a marked accumulation of glucose metabolites was observed. This shows that this combination reduces the glucose uptake and also the expulsion of end products, mainly lactic acid.

The glucose metabolites were identified by thin layer chromatography of the samples described above. After extraction of the cells in boiling water, the extracts were applied on TLC sheets. Standards containing glucose-6-P, fructose 1-6 diP, lactate, and PEP were included. PEP was present in all the samples, but in markedly higher amounts in the samples to which zinc chloride, NaF and xylitol were added. The content of hexose phosphate was also higher in these samples.

These results show that a preparation containing NaF, $ZnCl_2$, and xylitol inhibits the uptake of glucose in bacterial cells, and at the same time inhibits the metabolism of glucose which might have entered the cells The experiments support that the combination of fluorine compounds, zinc ions releasing compounds, and xylitol has a synergistic effect, and that this preparation is very effective in the suppression of bacteria which cause dental caries.

The effect of the combination of the three ingredients is markedly higher than the effect of the individual components or two-component combinations of them.

Further experiments also have shown that the combination according to the invention has a similar effect on dental plaque in vivo; a markedly reduced pH-drop was also observed compared with the effect of zinc salt, fluoride, or xylitol alone.

The compositions for oral hygiene according to the invention may be used in various application forms.

Toothpastes, either opaque or gel-like transparent, mouthwashes, and chewing gum are preferred; however, any other application form like mouth spray, sucking or chewing tablets, or tooth powder is suitable for this purpose.

A toothpaste may be opaque or transparent. Transparent toothpastes contain polishing agents having the same refraction index as the carrier material.

Especially suitable polishing agents are alumina, especially as trihydrate, such as $\alpha$-alumina trihydrate, having a preferred particle size distribution between about 1 and about 20, especially about 10 $\mu$m, and calcium carbonate.

However, it is also possible to use toothpastes based on other carrier materials containing as polishing agents e.g. alkali aluminium silicates, e.g. zeolites A as disclosed in European Published Patent Applications Nos. 2,690 and 3,023, different calcium phosphates like dicalcium orthophosphate as dihydrate or water-free, tricalcium phosphate, calcium pyrophosphate, insoluble alkali metaphosphates, silicas of different modifications, such as silica xerogels, hydrogels or precipitated silica, or powdered plastic materials like polymethyl methacrylate with a particle size distribution between about 0,5 and about 5 $\mu$m.

Silicas are preferred as suitable abrasive components.

Of course, also mixtures of the mentioned polishing substances may be used, e.g. a mixture of $\alpha$-alumina hydrate and/or calcium carbonate and synthetic zeolite A in a ratio of about 1:1.

The percentage of polishing agent in a toothpaste according to the invention is preferably between about 20 and about 60 % by weight of the total composition.

Of course, it is also possible to use the usual surface-active agents in toothpastes in amounts of up to 2,5 % by weight of the total composition.

Suitable synthetic surface-active agents are e.g. alkyl sulfates, alkyl ether sulfates, olefin sulfonates, sodium lauroyl sarcosinate, or ampholytic, nonionic, or cationic compounds or soaps like alkali salts from lauric acid, myristic acid, palmitic acid, stearic acid, or mixtures thereof, e.g., coconut oil fatty acids or tallow fatty acids.

Also essential constituents of toothpastes are moisturizers normally present in amounts between about 10 and about 35 % by weight. Suitable moisturizers are glycerol, diols like 1,4-butanediol or 1,2-propanediol or sugar alcohols like sorbitol, and also polyethylene glycols with low molecular weights.

Also contained in toothpastes are thickening agents, whose amount is between about 0,25 and about 5 % by weight of the total composition.

Preferred thickening agents are carboxymethyl cellulose and its alkali salts, especially sodium carboxymethyl cellulose, hydroxyalkyl celluloses like hydroxymethyl cellulose and hydroxyethyl cellulose, methyl cellulose, natural gums like tragant, Gum arabicum, Caraya gum, Guar gum, Xanthan gum, and Irish moss, synthetic polyelectrolytes like alkali salts of polyacrylic acid as well as inorganic thickening agents, especially colloidal magnesium aluminium silicate or colloidal silica.

The compositions for oral hygiene according to the invention may of course also contain additional active ingredients.

Those additional materials are further plaque-inhibiting substances such as copper salts, substances preventing the formation of dental calculus, such as hydroxyethane 1,1-diphosphonic acid or alkylene diamino tetramethylene phosphonic acids, and their water-soluble salts, allantoin, azulen, etc..

The synergistic three-component mixture of active ingredients contains preferably from about 0,1 to about 10, particularly from 0,5 to 7,5, especially from about 1 to about 5 % by weight of xylitol, calculated to the total composition for oral hygiene.

The amount of fluorine compound is preferably between about 0,01 and about 1, particularly 0,025 to 0,5, especially 0,05 to 0,15 % by weight, calculated to fluoride ($F^-$) ions, of the total composition.

Suitable fluorine compounds are the different salts of monofluorophosphoric acid such as sodium, potassium, lithium, calcium, and aluminum mono-and difluorophosphate as well as the various ionic fluorides, particularly alkali fluorides like sodium, lithium, potassium, and ammonium fluoride, stannous fluoride, manganese fluoride, copper fluoride, zirconium fluoride, and aluminum fluoride as well as mixtures or adducts of these fluorides, e.g. alkali manganese fluorides. Sodium fluoride and sodium monofluorophosphate are preferred.

The zinc ions releasing compounds are preferably present in an amount from about 0,1 to about 1 %, preferably 0,025 to 0,5 %, by weight, calculated to zinc ions of the total composition.

Generally all physiologically comparable zinc salts are suitable, such as zinc chloride, zinc fluoride, zinc fluosilicate, zinc citrate, zinc sulfate, zinc acetate, zinc formate, zinc butyrate, zinc caproate, zinc lactate, zinc valerate, zinc salicylate, zinc salts of amino acids such as zinc aspartate and zinc glycinate, zinc benzoate, zinc glycerophosphate, etc..

A review of components to be used in toothpastes as well as of other materials usually applied for the preparation of dental care agents and the manufacturing methods for these compositions are given in the monography of M.S. Balsam and E. Sagarin, "Cosmetics - Science and Technology", 2nd Ed., Vol. 1, p. 423 to 533 (1972), which is included by reference.

The following examples show compositions for oral hygiene according to the invention:

1. Toothpaste $\alpha$-Alumina trihydrate    35,00 (% by weight)
Zinc chloride ($ZnCl_2$)    1,00
Sodium monofluorophosphate    0,75

Xylitol    5,00
Sorbitol    10,00
Xanthan Gum    0,75
Sodium lauryl sulphate    1,20
Preservatives    0,35
Saccharin sodium    0,12
Flavour mixture    1,00
Dyes    0,01
Water    ad 100,00

2. Translucent toothpaste Silica Xerogel    17,00
(3-10 µm; 700 m²/g)
Silica Aerogel    1,50
Zinc citrate . 2 H₂O    1,10
Sodium fluoride    0,33
Xylitol    3,00
Sorbitol    20,00
Glycerol    15,00
Polyglycol 600    1,50
Xanthan Gum    1,00
Sodium lauroyl sarcosinate    1,20
Sodium saccharinate    0,10
Blue dye    0,01
Flavour mixture    1,00
Preservatives    0,30
Water    ad 100,00

3. Toothpaste Dicalcium orthophosphate    30,00
(% by weight)
(CaHPO₄ . 2H₂O)
Colloidal silica    2,20
Water-insoluble sodium metaphosphate    3,00
Irish Moss    0,90
Sodium lauryl sulfate    1,05
Sodium lauryl sarcosinate    0,50
Flavour mixture    1,00
Sodium cyclamate    0,50
Saccharin sodium    0,70
Zinc acetate . 2H₂O    0,50
Zinc chloride    0,70
Sodium monofluorophosphate    1,00
Xylitol    4,50
Sorbitol    11,50
Preservatives    0,25
Water    ad 100,00

4. Toothpaste Polymethyl methacrylate    30,00
powder ( 1-10 µm)
Precipitated silica    3,00
( 3-8 µm)
Zinc chloride    1,30
Sodium fluoride    0,33
Xylitol    3,80
Sorbitol    8,00
Glycerol    6,00
Sodium cocos oil sulfate    1,30
Magnesium aluminum sulfate    0,50
Carragheenan    0,50

Flavour mixture    1,20
Saccharine sodium    0,10
Preservatives    0,30
Water    ad 100,00

5. Mouthwash Zinc chloride (ZnCl₂)    0,20 (% by weight)
Sodium fluoride    0,10
Xylitol    2,00
Flavour mixture    4,00
Preservatives    0,25
Glycerol    7,50
Nonionic emulsifier    1,70
Ethanol    30,00
Water    ad 100,00

Before application for mouth rinsing, the above referred mouthwash is diluted with water in a ratio of 4:1.

6. Chewing gum Zinc aspartate    1,00
Zinc chloride    0,80
Xylitol    10,00
Sorbitol    25,00
Sodium fluoride    0,30
Flavour mixture    4,50
Gum base    ad 100,00

7. Toothpaste α-Alumina trihydrate    35,00 (% by weight)
Polyethene powder    5,00
Zinc sulfate    0,60
Zinc citrate . 2H₂O    0,60
Sodium monofluorophosphate    0,76
Sodium fluoride    0,11
Xylitol    2,50
Sorbitol    12,50
Sodium lauroyl sarcosinate    1,30
Flavour mixture    1,25
Saccharin sodium    0,12
Carragheenan    0,85
Magnesium aluminum silicate    0,45
Preservatives    0,30
Green dye solution    0,08
(1 % aqueous solution)
Water    ad 100,00

**Claims**

1. Composition for oral hygiene, containing a mixture of

(a) at least one fluorine compound,

(b) at least one zinc ions releasing compound,

(c) xylitol.

2. Composition according to claim 1, containing a mixture of

(a) at least one fluorine compound in an amount from 0,01 to 1,0 % by weight, calculated to fluoride ions;

(b) at least one zinc ions releasing compound in an amount from 0,01 to 2,0 % by weight, calculated to zinc ions;

(c) from 0,1 to 10,0 % by weight xylitol, all percentages calculated to the total composition.

3. Composition according to claim 2, containing the fluorine compound(s) in an amount from 0,025 to 0,5 % by weight, calculated to fluoride ions, of the total composition.

4. Composition according to claim 3, containing the fluorine compound(s) in an amount from 0,05 to 0,15 % by weight, calculated to fluoride ions, of the total composition.

5. Composition according to one or more of the preceding claims, containing the zinc ions releasing compound(s) in an amount from 0,1 to 1,0 % by weight, calculated to zinc ions, of the total composition.

6. Composition according to claim 5, containing the zinc ions releasing compound(s) in an amount from 0,015 to 0,5 % by weight, calculated to zinc ions, of the total composition.

7. Composition according to one or more of the preceding claims, containing from 0,1 to 10 % by weight xylitol, calculated to the total composition.

8. Composition according to claim 7, containing from 0,5 to 5,0 % by weight xylitol, calculated to the total composition.

9. Composition according to one or more of the preceding claims, being composed as a toothpaste, containing a mixture of

(a) 0,5 to 2,0 % by weight zinc chloride and (or) zinc acetate;

(b) 0,1 to 0,4 % by weight sodium fluoride and (or) 0,4 to 1,5 % by weight sodium mon-ofluorophosphate;

(c) 0,5 to 5,0 % by weight xylitol, calculated to the total composition.

Fig. 1

Intracellular $^{14}$C-glucose metabolites

Fig. 2

Intracellular $^{14}$C-glucose metabolites

Fig. 3